# EUROPEAN PATENT APPLICATION

(11) **EP 4 656 736 A2**
(43) Date of publication of application: **03.12.2025**
(21) Application number: 25189581.9
(22) Date of filing: 11.10.2017
(51) Int. Cl.: C12Q 1/6881

(54) **DETERMINING CELL TYPE ORIGIN OF CIRCULATING CELL-FREE DNA WITH MOLECULAR COUNTING**

(30) Priority: 12.10.2016 US 201662407408 P
(62) Divisional of application: 17860451.8
(71) Applicant: Bellwether Bio, Inc., Seattle, Washington 98115 (US)
(72) Inventor: SNYDER, Matthew William, Seattle, WA 98107 (US)
(74) Representative: Carpmaels & Ransford LLP

(57) **Abstract**

Provided herein are compounds, methods, and compositions for use in determining the cellular origin of circulating cell-free DNA.

## Description

### FIELD

Provided herein are compounds, methods, and compositions for use in determining the cellular origin of circulating cell-free DNA.

### BACKGROUND

Cell-free DNA (cfDNA) is present in the circulating plasma, urine, and other bodily fluids of humans. The cfDNA comprises both single- and double-stranded DNA fragments that are relatively short (overwhelmingly less than 200 base-pairs) and are normally at a low concentration (e.g. 1-100 ng/mL in plasma). In the circulating plasma of healthy individuals, cfDNA is believed to primarily derive from apoptosis of blood cells, i.e. normal cells of the hematopoietic lineage. However, in specific situations, other tissues can contribute substantially to the composition of cfDNA in bodily fluids such as circulating plasma. This fact has been exploited in recent years - in conjunction with the emergence of new technologies for highly cost-effective DNA sequencing - towards the development of novel clinical diagnostics in at least three areas.
1) Reproductive medicine: In pregnant women, a proportion of cfDNA in circulating plasma derives from fetal or placental cells containing the fetal genome (median 14%; range 2-60%, increasing with gestational age but highly variable between pregnancies(Bianchi, D.W. 2004. Placenta 25 Suppl A:S93-S101). Screening for genetic abnormalities in the fetus such as chromosomal trisomies can be achieved by deep sequencing of DNA libraries derived from circulating plasma cfDNA of a pregnant mother, a mixture of cfDNA derived from the maternal and fetal genomes. For example, if the fetus has trisomy 21, one expects to observe an excess of sequence reads mapping to chromosome 21 in sequencing of maternal plasma cfDNA. As this test has demonstrated major advantages with respect to sensitivity and specificity over other non-invasive screening tests, non-invasive aneuploidy screening based on analysis of circulating cell-free DNA is now routinely offered to women with high-risk pregnancies.
2) Cancer diagnostics: In cancer, a proportion of cfDNA in circulating plasma can derive from the tumor (with the % contribution from the tumor increasing with cancer stage but highly variable between individuals and cancer types). Cancer is caused by abnormal cells exhibiting uncontrolled proliferation secondary to mutations in their genomes. The observation of these mutations in circulating plasma cfDNA - a mixture of cfDNA derived from normal cells and cancer cells - has substantial promise to effectively serve as a "liquid biopsy" - for example, to non-invasively monitor for tumor recurrence. The types of mutations in a cancer genome that can be detected in this way include small mutations, e.g. a change to a single base-pair, as well as copy number alterations, e.g. copy gain or copy loss of one or more large regions or entire chromosomes.
3) Transplant medicine: After a transplant is performed, from a donor to a recipient, the major risk to the recipient is allograft rejection. A major clinical challenge is determining whether and the extent to which rejection is occurring, and the gold standard method for assessing rejection for many types of solid organ transplants involves invasive biopsy. Recently, the presence and abundance of circulating plasma cfDNA derived from the donor has been explored as a non-invasive marker for detecting and monitoring allograft rejection. For example, for female recipients of a solid organ transplant from a male donor, cfDNA derived from the Y chromosome unambiguously derives from the allograft and can be quantified. More generally, donor-specific genotypes - for example, determined by genome-wide genotyping of common variants or whole genome sequencing of both donor and recipient - can be exploited to differentiate between donor-derived and recipient-derived molecules when performing deep sequencing of cfDNA from circulating plasma or other bodily fluids of a transplant recipient.

There are several shared characteristics of the above-described clinical diagnostic tests. First, each test relies on sequencing of cfDNA, generally from circulating plasma but potentially from other bodily fluids. The sequencing is usually 'shotgun', but in some implementations is targeted to particular regions of the human genome. Second, the cfDNA that one is sequencing is typically derived from two or more cell populations bearing genomes that differ from one another with respect to primary nucleotide sequence and/or copy number representation of particular sequences (e.g. maternal genome vs. fetal genome; normal genome vs. cancer genome; transplant recipient genome vs. transplant donor genome). Third, the basis for each test is to either detect or monitor these genotypic differences between the two or more cell populations that contribute to the composition of cfDNA (e.g. fetal trisomy 21; cancer-specific somatic point mutations or aneuploidies: transplant donor-specific genotypes).

Although it is the basis for their success, the reliance of these cfDNA tests on genotypic differences nonetheless represents a major limitation. First, for all of these tests, the overwhelming majority of cfDNA molecules correspond to regions of the human genome where the two or more cell populations that one is trying to distinguish are identical at the sequence level. Consequently, in applications where one is quantifying cfDNA molecules that unambiguously derive from a specific cell population based on cell-type specific genotype(s), the vast majority of sequencing reads are uninformative. In applications where one is predicting the copy number content of one of the cell populations based on relative coverage of genomic regions (e.g. detecting trisomies from sequencing of maternal circulating cfDNA), a higher depth of sequencing coverage is required than if the origin of individual cfDNA molecules was knowable, or at least could be assigned non-uniform probabilities. Second, there are numerous pathologies wherein tissue damage or inflammatory processes are taking place and the tissue-of-origin composition of cfDNA might be expected to be altered as a consequence. However, these cannot always be detected by focusing on the genotypic differences between the contributing cell populations, simply because their genomes are identical or nearly identical. These include, for example, myocardial infarction (acute damage to heart tissue) and autoimmune disease (chronic damage to diverse tissues). However, they potentially also include many of the conditions described above such as cancer. For example, it has been observed that there is a major increase in the concentration of circulating plasma cfDNA in cancer, possibly disproportionate to the contribution from the tumor itself. This suggests that other tissues (e.g. stromal, immune system) may be contributing to circulating plasma cfDNA during cancer. However, these cell types have essentially unmutated genomes compared to the tumor, and as such cannot be readily distinguished from the cell types that normally contribute to cfDNA (e.g. normal cells of the hematopoietic lineage) based on genotypic differences.

Previously, cfDNA molecules that carry an epigenetic signature of their cell type of origin were identified, as evidenced in aggregate by the genomic coordinates of the millions of enzymatic fragmentation events giving rise to cfDNA during cell death. It was determined that nucleosomes, protein complexes called that contact DNA and are critical for its packaging in the nucleus, confer preferential protection to the specific base-pairs of DNA they contact. It was proposed that base-pairs contacted by nucleosomes are occluded from enzymes responsible for DNA degradation, thereby increasing the probability that these base-pairs will be observed, in aggregate, as members of plasma-borne cfDNA fragments. It was further demonstrated that the genomic endpoints of cfDNA fragments preferentially fall within short stretches of DNA - sometimes called "linkers" -- between adjacent nucleosomes, owing to preferential accessibility of these regions to enzymes responsible for digestion of chromatin.

These findings were paired with the observation that the genomic locations of a plurality of these nucleosomes vary between cell types and tissues. Knowledge of these nucleosome positions in a variety of cell types, obtained using orthogonal methods, were used to statistically model the cell types giving rise to cfDNA in a biological sample. Specifically, the following were measured: (a) the distribution of likelihoods any specific base-pair in a human genome will appear at a terminus of a cfDNA fragment (*i.e.* points of fragmentation); (b) the distribution of likelihoods that any pair of base-pairs of a human genome will appear as a pair of termini of a cfDNA fragment (*i.e.* consecutive pairs of fragmentation points that give rise to an individual cfDNA molecule): and (c) the distribution of likelihoods that any specific base-pair in a human genome will appear in a cfDNA fragment (*i.e.* relative coverage) as a consequence of differential nucleosome occupancy, comparing two or more cell types.

Finally, it was demonstrated that these findings could inform clinical decision making in the context of one or more physiological conditions. The anatomical origin of the primary tumor could be identified using the described methods in at least some human cancers, which could inform therapeutic interventions or other treatment options.

The methods described above enable the determination and quantification of the relative contributions of cell types to the population of cfDNA in a biological sample on the basis of the combination of the manipulation of the purified cfDNA, the comparison to reference nucleosome maps, and one or more statistical techniques to enable this comparison. However, nucleosomes are not the only proteins that confer protection to DNA during the process of cfDNA genesis and provide cell-type information. Different cell types within and between tissues make use of a different complement of transcription factors (TFs), proteins that bind specific regions of DNA and control or regulate expression of one or more genes. Some TFs are specific to single cell types, others are shared across all cell types, and still others are utilized by multiple cell lineages to different extents (e.g. in different concentrations across cell types). The occupancy of these TFs occludes the occupied base-pairs from enzymatic access, conferring protection from degradation during cell death in a way that is related to the protection conferred by nucleosomes.

The number of base-pairs occupied and protected by a single TF is typically much lower than the number of base-pairs occupied and protected by a single nucleosome. Nucleosomes confer approximately 150 base-pairs of protection; TFs, on the other hand, confer protection to tens of base-pairs - typically 10-50 base-pairs, depending on the specific TF. This protected region of DNA is often called the "footprint" of the TF. In general, TFs exhibit sequence specificity, such that they preferentially bind segments of DNA with stereotyped sequences of nucleotides (*e.g.*, TCGGATCTTC). In some cases, the sequences preferred by a TF may be degenerate.

After sequencing of cfDNA and appropriate quality control of the resulting "reads" (the ordered list of nucleotides comprising each sequenced molecule), a conventional next step in many workflows is mapping these reads to a reference genome. The process of mapping identifies the genomic origin of each fragment on the basis of a sequence comparison. In many cases, long reads - reads composed of 100 nucleotides or more -- can be confidently mapped to unique genomic locations, as the probability of exact matches occurring more than once in the reference genome decays as the length of the reads increases. Conversely, as reads get shorter, the probability of multiple, equally good mapping locations increases. In many workflows, reads that cannot be confidently mapped to one and only one genomic origin are discarded.

In many cases, the short sequences of DNA that are preferred by a specific TF reoccur multiple times throughout the reference genome. In this manner, a single TF can bind the DNA in these multiple locations, where it can then play a role in regulation of nearby genes. Experiments to determine the footprints of a wide variety of TFs have been performed, and the many genomic locations of these sequences can be quickly identified using a variety of computational techniques.

In sequencing data derived from biological cfDNA samples, a variable proportion of reads is derived from TF protection, and these reads are thus short - approximately 10-50 bases. Many of these reads cannot be confidently uniquely mapped, and are thus discarded in many workflows.

### SUMMARY

Provided herein are compounds, methods, and compositions for use in determining the cellular origin of circulating cell-free DNA.

In certain embodiments, the methods provided herein are useful in determining the cellular origin of circulating cell-free DNA. In advantageous embodiments, the methods are useful in determining cellular origin where cell-free DNA can originate from a plurality of hosts, for instance, in a pregnant individual.

### DESCRIPTION OF EXEMPLARY EMBODIMENTS

Provided herein are compounds, methods, and compositions for use in determining the cellular origin of circulating cell-free DNA. In certain embodiments, provided herein are methods for determining or quantifying the cell types and tissue-of-origin composition of cfDNA in bodily fluids on the basis of transcription factor (TF) footprints in short cfDNA fragments.

In particular embodiments of the methods, cell-free DNA (cfDNA) is extracted and purified from a source. Extraction and purification can proceed according to techniques known to those of skill in the art. For example, the QIAGEN QIAamp Circulating Nucleic Acid kit is a common method, based on the binding of cfDNA to a silica column, for purification of cfDNA from plasma or urine. An alternative method, phenol-chloroform extraction followed by isopropanol or ethanol precipitation, provides similar results while allowing for more flexibility in the volume of the biological sample.

After purification of cfDNA from biological fluids, the fragments can be subjected to one or more enzymatic steps to create a sequencing library. The enzymatic steps can proceed according to techniques known to those of skill in the art. An example of these enzymatic steps is described in Kivioja et al. (2011. Nat Methods 9(1):72-74). Commercial products such as Rubicon Genomics' ThruPLEX Tag-seq are also used to create sequencing libraries from purified cfDNA.

According to the methods, the cfDNA fragments are tagged with an oligonucleotide unique molecular identifier (UMI) to facilitate identification of unique fragments. The UMI is typically a DNA oligomer. In certain embodiments, the UMI has a random sequence. In certain embodiments, the UMI is approximately 3-10 base-pairs in length. The UMI can serve as a molecular barcode.

Library amplification (*e.g.* with PCR) and sequencing can each result in the same original cfDNA fragment being sequenced more than once and thus appearing as duplicate reads. However, cfDNA fragments may also be truly biologically duplicated at the sequence level - a possibility that is magnified as fragment length decreases. Disentangling these two scenarios - true biological duplication and technical duplication - is difficult or impossible with conventional DNA sequencing workflows. However, the addition of a UMI to each molecule allows these two scenarios to be disentangled, by uniquely tagging each molecule to allow the identification of technical duplicates (which would carry the same UMI).

In certain embodiments, the UMI-tagged cfDNA fragments are amplified. Amplification can proceed according to techniques known to those of skill in the art. In certain embodiments, the UMI-tagged cfDNA fragments are sequenced. Sequencing can proceed according to techniques known to those of skill in the art.

Following sequencing, duplicates can be identified by comparing reads on the basis of both their UMIs and their genomic locations and/or sequences. Technical duplicates, which share genomic locations and/or sequences as well as UMIs, can be discarded. Biological duplicates, which share genomic locations and/or sequences but do not share UMIs, can be retained. These remaining sequences can then be partitioned into length classes to enrich for TF footprints in the shortest class(es).

In certain embodiments, the reads that cannot be uniquely mapped are separated from the reads that can be uniquely mapped. These reads can be computationally compared to existing compendia of TF footprints (also known as "motifs") to identify TFs that are likely to have conferred protection to the fragments from which the reads were derived. The comparison to existing compendia does not require exact sequence matches. In some embodiments, one or more sequence mismatches can be allowed to account for imperfect sequence specificity on the part of the TF. In some embodiments, the comparison is performed by searching for one or more informative subsequences of length *k* (often called "k-mers"), with gaps ("gapped k-mers) or without gaps. The number of such reads derived from each TF using this comparison is tallied by counting the UMIs, thus allowing the relative frequency of each TF's footprint in the sample of reads to be quantified. By iterating this procedure across a large number of TFs, a vector of TF frequencies can be populated for each biological sample. This vector can then be normalized across biological samples and sequencing datasets (e.g. from multiple individuals, or from the same individual over time) by comparing to counts of uniquely mapped reads within a predefined set of genomic loci in each sample (i.e., accounting both for sequencing coverage and for fragment length biases owing to technical differences between samples).

In some embodiments, the vector of counts for each TF is then modeled as a mixture of TF profiles found in myriad cell types using orthogonal methods, including ChIP-seq assays such as those performed by the ENCODE project. This modeling can have several embodiments. In one embodiment, the comparison involves a computational search for TF footprints that are present in the biological sample and whose cognate TFs are specific to a single cell type. In another embodiment, the vector of molecular counts described above and derived from a biological sample is modeled as a linear combination of vectors of TF profiles derived from orthogonal methods. The output from each embodiment is a list of contributing cell types, optionally including estimated proportions for each contributor in some embodiments.

Transcription factor utilization is a dynamic process, such that single cells of the same type are not identical with respect to TF occupancy along their genome. Nonetheless, at the aggregate level, the complement of TFs within a cell is known to be cell type-specific. In other words, there are many coordinates in the genome at which the probability of TF occupancy substantially differs between cell or tissue types.

The methods provided herein are based, at least in part, on the discovery that short cfDNA fragments, despite typically being discarded because their length challenges unique genomic placement, contain information about the complement of TFs active upon cell death and cfDNA genesis. The addition of unique molecular identifiers enables counting-based relative quantification of these TFs, and can be used to differentiate the relative contributions of two or more tissue or cell types to the composition of cfDNA in bodily fluids. Furthermore, the comparison of TF profiles between individuals and/or samples can be used to diagnose and/or monitor any pathology or clinical conditions in humans in which the tissue-of-origin composition of cfDNA in bodily fluids is substantially altered in a way that consistently correlates with that pathology or clinical condition.

All publications and patent, applications cited in this specification are herein incorporated by reference as if each individual publication or patent application were specifically and individually indicated to be incorporated by reference. While the claimed subject matter has been described in terms of various embodiments, the skilled artisan will appreciate that various modifications, substitutions, omissions, and changes may be made without departing from the spirit thereof. Accordingly, it is intended that the scope of the subject matter limited solely by the scope of the following claims, including equivalents thereof.

**The invention is further characterized by the following numbered non-limiting embodiments:**
1. A method of determining tissues and/or cell types giving rise to cell-free DNA (cfDNA) in a subject, the method comprising:
   a. isolating cfDNA from a biological sample from the subject, the isolated cfDNA comprising a plurality of cfDNA fragments;
   b. tagging a unique molecular identifier (UMI) to each isolated cfDNA fragment, the UMI comprising an oligomer of at least two nucleotides;
   c. determining pairs of sequences associated with at least a portion of the plurality of UMI-tagged cfDNA fragments;
   d. determining the subset of these pairs of sequences for which the sequence associated with the cfDNA fragment has more than one genomic location within a reference genome; and
   e. determining at least some of the tissues and/or cell types giving rise to the cfDNA fragments as a function of this subset of pairs of sequences.
2. The method of embodiment 1 wherein the step of determining at least some of the tissues and/or cell types giving rise to the cfDNA fragments comprises comparing the sequences associated with the cfDNA fragments to one or more reference maps.
3. The method of embodiment 2 wherein the reference maps comprise binding motifs for at least one transcription factor.
4. The method of embodiment 2 wherein the reference maps comprise binding locations for at least one transcription factor.
5. The method of embodiment 4 wherein the binding locations for at least one transcription factor are determined by immunoprecipitation (*e.g.* with ChIP-seq)
6. The method of any preceding embodiment further comprising generating a report comprising a list of the determined tissues and/or cell types giving rise to the isolated cfDNA.
7. A method of identifying a disease or disorder in a subject, the method comprising:
   a. isolating cfDNA from a biological sample from the subject, the isolated cfDNA comprising a plurality of cfDNA fragments;
   b. tagging a unique molecular identifier (UMI) to each isolated cfDNA fragment, the UMI comprising an oligomer of at least two nucleotides;
   c. determining pairs of sequences associated with at least a portion of the plurality of UMI-tagged cfDNA fragments;
   d. determining the subset of these pairs of sequences for which the sequence associated with the cfDNA fragment has more than one genomic location within a reference genome;
   e. determining at least some of the tissues and/or cell types giving rise to the cfDNA fragments as a function of this subset of pairs of sequences; and
   f. identifying the disease or disorder as a function of the determined tissues and/or cell types giving rise to the cfDNA.
8. The method of embodiment 7 wherein the step of determining at least some of the tissues and/or cell types giving rise to the cfDNA fragments comprises comparing the sequences associated with the cfDNA fragments to one or more reference maps.
9. The method of any preceding embodiment wherein the reference genome is associated with a human.
10. The method of any preceding embodiment comprising generating a report comprising a statement identifying the disease or disorder.
11. The method of embodiment 10 wherein the report further comprises a list of the determined tissue(s) and/or cell type(s) giving rise to the isolated cfDNA.
12. The method of any preceding embodiment wherein the biological sample comprises, consists essentially of, or consists of whole blood, peripheral blood plasma, urine, or cerebral spinal fluid.
13. The method of either of embodiment 1 or 7 wherein the step of determining at least some of the tissues and/or cell types giving rise to the cfDNA fragments comprises counting UMIs associated with identical cfDNA sequences to produce a vector of counts.
14. The method of embodiment 13 wherein each UMI is tallied only once regardless of the number of times it appears in the subset.
15. The method of embodiment 13 wherein the step of determining at least some of the tissues and/or cell types giving rise to the cfDNA fragments comprises performing a mathematical transformation on the vector of counts.

## Claims

1. A method of determining tissues and/or cell types giving rise to cell-free DNA (cfDNA) in a subject, the method comprising:
a. isolating cfDNA from a biological sample from the subject, the isolated cfDNA comprising a plurality of cfDNA fragments;
b. tagging a unique molecular identifier (UMI) to each isolated cfDNA fragment, the UMI comprising an oligomer of at least two nucleotides;
c. determining pairs of sequences associated with at least a portion of the plurality of UMI-tagged cfDNA fragments;
d. determining the subset of these pairs of sequences for which the sequence associated with the cfDNA fragment has more than one genomic location within a reference genome; and
e. determining at least some of the tissues and/or cell types giving rise to the cfDNA fragments as a function of this subset of pairs of sequences.

2. The method of claim 1 wherein the step of determining at least some of the tissues and/or cell types giving rise to the cfDNA fragments comprises comparing the sequences associated with the cfDNA fragments to one or more reference maps.

3. The method of claim 2 wherein the reference maps comprise binding motifs for at least one transcription factor.

4. The method of claim 2 wherein the reference maps comprise binding locations for at least one transcription factor.

5. The method of claim 4 wherein the binding locations for at least one transcription factor are determined by immunoprecipitation (e.g. with ChIP-seq)

6. The method of any preceding claim further comprising generating a report comprising a list of the determined tissues and/or cell types giving rise to the isolated cfDNA.

7. A method of identifying a disease or disorder in a subject, the method comprising:
a. isolating cfDNA from a biological sample from the subject, the isolated cfDNA comprising a plurality of cfDNA fragments;
b. tagging a unique molecular identifier (UMI) to each isolated cfDNA fragment, the UMI comprising an oligomer of at least two nucleotides;
c. determining pairs of sequences associated with at least a portion of the plurality of UMI-tagged cfDNA fragments;
d. determining the subset of these pairs of sequences for which the sequence associated with the cfDNA fragment has more than one genomic location within a reference genome;
e. determining at least some of the tissues and/or cell types giving rise to the cfDNA fragments as a function of this subset of pairs of sequences; and
f. identifying the disease or disorder as a function of the determined tissues and/or cell types giving rise to the cfDNA.

8. The method of claim 7 wherein the step of determining at least some of the tissues and/or cell types giving rise to the cfDNA fragments comprises comparing the sequences associated with the cfDNA fragments to one or more reference maps.

9. The method of any preceding claim wherein the reference genome is associated with a human.

10. The method of any preceding claim comprising generating a report comprising a statement identifying the disease or disorder.

11. The method of claim 10 wherein the report further comprises a list of the determined tissue(s) and/or cell type(s) giving rise to the isolated cfDNA.

12. The method of any preceding claim wherein the biological sample comprises, consists essentially of, or consists of whole blood, peripheral blood plasma, urine, or cerebral spinal fluid.

13. The method of either of claim 1 or 7 wherein the step of determining at least some of the tissues and/or cell types giving rise to the cfDNA fragments comprises counting UMIs associated with identical cfDNA sequences to produce a vector of counts.

14. The method of claim 13 wherein each UMI is tallied only once regardless of the number of times it appears in the subset.

15. The method of claim 13 wherein the step of determining at least some of the tissues and/or cell types giving rise to the cfDNA fragments comprises performing a mathematical transformation on the vector of counts.
